(19) Europäisches Patentamt
European Patent Office
Office européen des brevets

(11) Veröffentlichungsnummer: **0 025 190**
**B1**

(12) # EUROPÄISCHE PATENTSCHRIFT

(45) Veröffentlichungstag der Patentschrift: **13.06.84**

(21) Anmeldenummer: **80105113.7**

(22) Anmeldetag: **28.08.80**

(51) Int. Cl.³: **C 07 C 103/52,**
C 12 Q 1/38, C 12 Q 1/56

(54) **Chromogene Verbindungen und ihre Verwendung als Enzymsubstrate.**

(30) Priorität: **10.09.79 DE 2936543**

(43) Veröffentlichungstag der Anmeldung:
**18.03.81 Patentblatt 81/11**

(45) Bekanntmachung des Hinweises auf die Patenterteilung:
**13.06.84 Patentblatt 84/24**

(84) Benannte Vertragsstaaten:
**AT BE CH DE FR GB IT LI LU NL SE**

(56) Entgegenhaltungen:
**EP - A - 0 000 063**
**EP - A - 0 018 112**
**EP - A - 0 019 589**
**WO - A - 79/00602**
**DE - A - 2 629 067**
**DE - A - 2 649 171**
**DE - A - 2 740 323**
**FR - A - 2 183 188**
**FR - A - 2 279 106**
**US - A - 4 191 808**

(73) Patentinhaber: **BEHRINGWERKE AKTIENGESELLSCHAFT**
**Postfach 1140**
**D-3550 Marburg/Lahn (DE)**

(72) Erfinder: **Karges, Hermann Erich, Dr.**
**Sonnenhang 32**
**D-3550 Marburg/Lahn (DE)**
Erfinder: **Heber, Helmut, Dr.**
**Am Ziegenberg 8**
**D-3550 Marburg/Lahn (DE)**
Erfinder: **Uhmann, Rainer, Dr.**
**An der Landwehr 5**
**D-6239 Kriftel (DE)**
Erfinder: **Teetz, Volker, Dr.**
**An der Tann 20**
**D-6238 Hofheim am Taunus (DE)**
Erfinder: **Geiger, Rolf, Prof. Dr.**
**Heinrich-Bleicher-Strasse 33**
**D-6000 Frankfurt am Main 50 (DE)**

(74) Vertreter: **Meyer-Dulheuer, Karl-Hermann, Dr. et al,**
**HOECHST Aktiengesellschaft Zentrale**
**Patentabteilung Postfach 80 03 20**
**D-6230 Frankfurt/Main 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

(56) Entgegenhaltungen:
**EXPERIENTIA, Band 34, Nr. 3, März 1978**
**Birkhäuser Verlag BASEL (CH) T. KATO et al.:**
**"Studies on Substrate Specificity of X-Prolyl**
**Dipeptidyl-aminopeptidase using new**
**chromogenic substrates, X-Y-p-nitroanilides"**
**Seiten 319-320**

**INTERNATIONALE VERÖFFENTLICHTUNG, WO -**
**A - 80/00351 (AM. HOSPITAL SUPPLY CO.)**

**Beschreibung**

Die Erfindung betrifft chromogene Verbindungen, die als Substrate zum Nachweis und zur Bestimmung von hydrolytisch wirkenden Enzymen geeignet sind.

Chromogene Substrate zur Aktivitätsbestimmung von Enzymen sind seit langem bekannt. So benützt man beispielsweise Benzoyl-arginyl-4-nitranilid, un die Trypsinwirkung eines Enzyms quantitativ zu erfassen, indem die pro Zeiteinheit freigesetzte Menge 4-Nitranilin gemessen wird. Diese Messung wird dadurch möglich, daß sich das UV-Spektrum des freien 4-Nitranilins von dem der N-acylierten Verbindung stark unterscheidet.

Ähnliche Substrate zur Bestimmung der Aktivität von Serinproteasen EC 3.421 sind beispielsweise aus den DE—A—25 27 932, 25 52 570, 26 29 067, 26 49 649 oder 27 40 323, WO—A—79/000 602, FR—A—2 183 188, FR—A—2 279 106, EP—A—0-000-063 sowie aus Experimenta, Band 34, Nr. 3, Seite 319, 1978 bekannt. Sie enthalten statt des Benzoylrestes freie oder N-geschützte Peptide aus 2—3 Aminosäuren. Die chromophore Gruppe ist auch hier meist 4-Nitranilin.

Diese Substrate nach dem Stand der Technik haben jedoch Mängel hinsichtlich der Spezifität. So können beispielsweise nach Thromb. Res. *10*, 549 (1977) solche Substrate nicht nur durch Proteasen, sondern von bestimmten Esterasen mit fast der gleichen Geschwindigkeit gespalten werden. Aufgabe der Erfindung ist es unter anderem, diesen Mangel zu beheben.

Den Nachteil der Unspezifität weisen die neuen Verbindungen bei ihrer Verwendung als erfindungsgemäße Enzymsubstrate in weit geringerem Maße als die Substrate des Standes der Technik auf. Während in den Substraten nach dem Stand der Technik stets eine nichtpeptidische Bindung zwischen einer basischen Aminosäure und den Chromogenen gespalten wird, liegt in den erfindungsgemäßen Peptiden an der Spaltstelle für die Enzyme eine echte Peptidbindung zwischen der basischen Aminosäure und einer anderen, natürlich vorkommenden Aminosäure vor. Die neuen Substrate kommen deshalb bei der enzymatischen Spaltung den Eigenschaften eines Proteins näher als die bisher bekannten Verbindungen: Die Esteraseempfindlichkeit ist durch das Einschieben einer oder mehrerer, Aminosäuren zwischen Spaltstelle und Chromophor stark reduziert, während die Spezifität durch eine Variation dieser Aminosäuren erhöht werden kann. Die Verwendung der neuen Verbindungen als chromogene beziehungsweise fluoreszierende Substrate zum Nachweis, vorzugsweise zur quantitativen Bestimmung von proteolytischen Enzymen, stellt im besonderen den Gegenstand der Erfindung dar. In bekannter Weise können die Verbindungen auch bei der Bestimmung von Enzyminhibitoren eingesetzt werden. Vor allem eignen sich die Verbindungen als chromogene Substrate bei der Bestimmung von Proteinasen in Körperflüssigkeiten von Menschen und Tieren.

Gegenstand der Erfindung sind Verbindungen der Formel I,

$$W—P—B—X—NH—R \qquad (I)$$

worin

- W ein Wasserstoffatom, eine Acylgruppe oder eine Benzolsulfonyl-oder Alkarylsulfonylgruppe mit 7—10 Kohlenstoffatomen,
- P einen Rest einer Aminosäure oder eines Di- bis Tetrapeptids, deren Seitenkettenfunktionen mit in der Peptidchemie üblichen Schutzgruppen substituiert sein können,
- B Arginin, Lysin, Phenylalanin, Tyrosin oder Homoarginin,
- X eine L-Aminosäure mit hydrophober Seitenkette oder ein hydrophobes Derivat einer natürlichen Aminosäure und
- R ein gegebenenfalls substituententragender aromatischer Kohlenwasserstoffrest bedeutet, wobei X—NH—R eine chromogene oder fluorogene Gruppe darstellt und die Eigenschaft besitzt, unter der hydrolytischen Einwirkung eines Enzyms ein Spalt-produkt $NH_2$—R abzugeben, dessen Menge durch photometrische, spektophotometrische oder fluorenzphotometrische Methoden meßbar ist.

Bevorzugte Bedeutungen sind:

Für W: eine $R_1$—CO-Gruppe, worin $R_1$ ein aliphatischer Kohlenwasserstoffrest von 1—6 Kohlenstoffatomen, ein aromatischer Kohlenwasserstoffrest mit 6—10 Kohlenstoffatomen, ein araliphatischer Kohlenwasserstoffrest mit 7—10 Kohlenstoffatomen, eine Alkaryl-Gruppe mit bis zu 10 Kohlenstoffatomen, eine $R_2$—O—CO-Gruppe, in welcher $R_2$ ein aliphatischer Kohlenwasserstoffrest mit 1—6 Kohlenstoffatomen, ein araliphatischer Kohlenwasserstoffrest mit 7—10 Kohlenstoffatomen oder eine Alkyl-6-sulfonalkyl$_{2-6}$-Gruppe bedeutet, eine Benzosulfonyl-Gruppe oder eine Alkarylsulfonyl-Gruppe mit 7—10 Kohlenstoffatomen;

Für R: eine Nitrophenyl-, Naphthyl-, Nitronaphthyl-, Methoxynaphthyl-, Phenyl-, Methylnitrophenyl-, Dinitrophenyl-, Chinolyl- oder Nitrochinolyl-Gruppe sowie deren Salze.

Besonders bevorzugte Bedeutungen sind:

Für R: ein Nitrophenylrest, der mit einem Halogenatom, einer Trifluormethylgruppe, einer Carboxygruppe, einer Methoxygruppe, einer Sulfosäuregruppe oder einer Cyanogruppe substituiert sein kann, eine Di-(methoxycarbonyl)-phenyl-Gruppe, ein 4-Azobenzol-, 4-Sulfonaphthalin-, Pyridin, Nitrobenzothiazol-, Pyrazin-, Triazol-, Benzopyrazol-, Chrysen-, Naphthalin-, Methoxynaphthalin-, Azulen- oder Aminocumarinrest ist.

Wenn in einer solchen Verbindung durch die Einwirkung eines nachzuweisenden oder zu bestimmenden Enzyms die Bindung B—X gespalten wird, entsteht ein chromogenes Aminosäurederivat H—X—NH—R. Um daraus den Chromophor bzw. Fluorophor $NH_2$—R freizusetzen, muß die Bindung X—N gespalten werden, wozu sich besonders ein Enzym mit Aminopeptidasewirkung eignet. Dabei ist eine möglichst höhe Spaltungsgeschwindigkeit, gute Stabilität des Enzyms in Lösung und evtl. Lyophilisierbarkeit erwünscht. Beispielsweise wurde in der Aminopeptidase K aus dem Pilz Tritirachium album Limber ein geeignetes Enzym mit den oben genannten Eigenschaften gefunden. Die Reaktionsfolge kann somit wie folgt beschrieben werden:

W – P – B – X – NH – R

↓ Protease

W – P – B – OH + H – X – NH – R

↓ Aminopeptidase

H – X – OH + $H_2$N – R (Chromophor, Fluorophor)

Die dargestellte Reaktionsfolge gibt gleichzeitig das Prinzip der Verwendung der erfindungsgemäßen Verbindung bei der Bestimmung einer Proteinase wieder: Die Proteinase spaltet das Substrat an einer spezifischen Bindungsstelle, wonach eine einzusetzende Aminopeptidase aus einem der Spaltprodukte das einem Meßverfahren zugängliche Chromophor abspaltet. Es stellt im besonderen den Gegenstand der Erfindung, die Verwendungen der Verbindungen der allgemeinen Formel I für die Bestimmung von hydrolytisch wirksamen Enzymen, dar. Diese betrifft ein Verfahren zur Bestimmung eines hydrolytisch wirksamen Enzyms, das dadurch gekennzeichnet ist, daß man die Lösung, in der das Enzym bestimmt werden soll, versetzt mit einer Verbindung der allgemeinen Formel I

worin

W ein Wasserstoffatom, eine Acylgruppe oder eine Benzolsulfonyl-oder Alkarylsulfonylgruppe mit 7—10 Kohlenstoffatomen,

P einen Rest einer Amonsäure oder eines Di- bis Tetrapeptids, deren Seitenkettenfunktionen mit in der Peptidchemie üblichen Schutzgruppe substituiert sein können, oder eine Bindung,

B Arginin, Lysin, Phenylalanin, Tyrosin oder Homoarginin,

X X eine oder zwei L-Aminosäuren mit hydrophober Seitenkette oder ein hydrophobes Derivat einer natürlichen Aminosäure, und

R einen gegebenenfalls substituententragenden aromatischen Kohlenwasserstoffrest bedeutet, wobei X—NH—R eine chromogene Gruppe darstellt und die Eigenschaft besitzt, under der hydrolytischen Einwirkung eines Enzyms ein Spaltprodukt $NH_2$—R abzugeben, dessen Menge durch photometrische, spektrophotometrische oder fluorenzphotometrische Methoden meßbar ist, einem Enzym mit Aminopeptidase-Wirkung und nach einer Inkubationszeit das Spaltprodukt $NH_2R$ der Verbindung der allgemeinen Formel I mißt.

Für die in der Verbindung der Formel I enthaltenen Aminosäuren gilt das im folgenden zu den Aminosäuren $A_1$ bis $A_4$ geschriebene entsprechend.

Das abgespaltene Chromophor bzw. Fluorophor ist ein direktes Maß für die Aktivität des hydrolytisch wirksamen Enzyms. Das Enzym mit Aminopeptidase-Wirkung stellt in diesem Verfahren ein Hilfsenzym dar. Hierfür sind besonders solche Enzyme mit Aminopeptidase-Wirkung geeignet, die aus tierischen oder humanen Geweben oder aus Mikroorganismen gewonnen werden. Beispielsweise sind hier Leucin-Aminopeptidase, Aminopeptidase, Aminopeptidase M oder das bereits genannte Enzym aus Tritirachium album Limber geeignet. Die erfindungsgemäßen Verbindungen lassen sich durch folgende Darstellung in der Formel II näher charakterisieren:

$$W—A_1—A_2—A_3—A_4—B—X—NH—R \quad (II)$$

Darin bedeuten $A_2$ bis $A_4$ je einen Rest einer $\alpha$-Amino- oder Iminosäuren, die gleich oder verschieden und deren Seitenkettenfunktionen substituiert sein können oder eine chemische Bindung. Falls es sich dabei um chirale Aminosäuren handelt, können sie in der L- oder D-Form vorliegen. Die Aminosäuren $A_2$ bis $A_4$ sind aber nicht nur auf solche beschränkt, die in Proteinen vorkommen. Auch $\alpha$-Aminosäuren wie z.B. $\alpha$-Aminoisobuttersäure (Aib), Pipecolinsäure, Azetidincarbonsäure, OMe-Tyrosin, 1-Aminocyclohexan-carbonsäure, Cysteinsäure, Phenylglycin (Phg), $\gamma$-Benzimidazolyl-aminobuttersäure, Spinacin und viele andere sowie $\beta$-Aminosäuren haben sich zur Erzielung hoher Enzymspezifität bewährt.

Tragen die Aminosäuren $A_2$—$A_4$ zusätzliche funktionelle Gruppen wie OH, COOH, $NH_2$, SH, so können diese frei oder geschützt vorliegen, beispielsweise geschütztes Arginin, Histidin, Asparagin, Glutamin. Eine Aminogruppe in der Seitenkette sollte immer acyliert sein, um als basische Aminosäure keine zusätzliche Spaltstelle zu liefern. Als Schutzgruppen kommen die in der Peptidchemie üblichen Reste in Frage (vgl. E. Wünsch in Houben-Weyl, Band XV 1/2) beispielsweise für Carboxylgruppen Ester oder Amide, für Hydroxy- und Mercaptogruppen bevorzugt Alkyl, Aryl- oder Aralkylgruppen, für Amine einfache Alkanoyl- oder Aroylreste, Kohlensäurehalbester, Toluolsulfonyl, Trifluoracetyl und andere.

In der Peptidchemie werden üblicherweise solche chemischen Gruppen als Schutzgruppe bezeichnet, die unter Bedingungen abgespalten werden können, unter denen Peptidbindungen nicht in wesentlichem Umfang verändert werden. Es besteht jedoch keine Notwendigkeit, aus den erfindungsgemäßen Verbindungen solche Schutzgruppe, beispielsweise die N-endständige Schutzgruppe W, abzuspalten, um sie für den erfindungsgemäßen Zweck verwenden zu können. Deshalb brauchen die in den erfindungsgemäßen Peptidderivaten vorhandenen Schutzgruppen die Bedingung der Abspaltbarkeit, ohne daß das Restmolekül geschädigt wird, nicht zu erfüllen. Es kommen deshalb als Schutzgruppen im Sinne der Erfindung weiterhin alle solchen chemischen Gruppierungen in Betracht, welche die in den erfindungsgemäßen Verbindungen endständige Aminogruppe oder die in den mit P und X bezeichneten Molekülteilen vorhandenen Seitenkettenfunktionen der Aminosäurereste während der Synthese dieser Verbindungen gegen eine unerwünschte chemische Reaktion zu schützen geeignet sind, auch wenn sie nicht aus den erfindungsgemäßen Verbindungen ohne Schaden für das Molekül abgespalten werden könnten. Eine N-endständige Schutzgruppe W oder eine Schutzgruppe in der Seitenkette einer der Aminosäuren in dem Molekülteil P oder X kann deshalb eine Acyl- oder Sulfonylgruppe, vorzugsweise eine $R_1$—CO-Gruppe, worin $R_1$ ein aliphatischer Kohlenwasserstoffrest von 1—6 Kohlenstoffatomen, der mit 1—3 Fluor-, Chlor-, Brom- oder Jodatomen oder Carboxygruppen substituiert sein kann, ein aromatischer Kohlenwasserstoffrest mit 6—10 Kohlenstoffatomen, der mit 1—3 Halogenatomen substituiert sein kann, ein aliphatischer Kohlenwasserstoffrest mit 7—10 Kohlenstoffatomen, eine Alkaryl-Gruppe mit 6—10 Kohlenstoffatomen, eine $R_2O$—CO-Gruppe, in welcher $R_2$ ein aliphatischer Kohlenwasserstoffrest mit 1—6 Kohlenstoffatomen, ein araliphatischer Kohlenwasserstoffrest mit 7—10 Kohlenstoffatomen oder eine Alkyl$_{1-6}$-Sulfonalkyl$_{2-6}$-Gruppe bedeutet, eine Benzolsulfonyl-Gruppe oder eine Alkarylsulfonyl-Gruppe mit 7—10 Kohlenstoffatomen, besonders auch Formyl (For), Acetyl (Ac), Benzoyl (Bz), Trifluoracetyl (TFA), Benzyloxycarbonyl (Z), tert. Butyloxycarbonyl (BOC), Methyloxycarbonyl (Meoc), Ethyloxycarbonyl (Etoc), Methylsulfonylethyloxycarbonyl (Msc), Toluolsulfonyl (Tos), Mesyl (Mes) oder Pyroglutamyl (pGlu) sein, ferner ein monoalkyliertes Polyethylenglycol, dessen freie OH-Gruppe über eine Ester-amid-, Urethan- oder Urethan-Ureid-Bindung mit $A_1$ verknüpft ist, Succinoyl (Suc) oder Maleoyl (Mal).

Für die Aminosäure $A_1$ gilt zunächst das für die Aminosäuren $A_2$—$A_4$ Gesagte. Steht W jedoch für Wasserstoff, muß $A_1$ für Aminopeptidasen unangreifbar oder zumindest schwer abbaubar sein. Dies gilt z.B. für natürlich vorkommende D-$\alpha$-Aminosäuren oder $\beta$-Aminosäuren wie $\beta$-Alanin oder auch andere Aminosäuren wie z.B. $\alpha$-Aminocapronsäure, Pyroglutaminsäure, Glycin oder Serin, insbesondere in Verbindung mit Prolin, Pipecolinsäure oder einer D-Aminosäure als $A_2$.

B ist L-Arginin, L-Lysin, L-Phenylalanin, L-Tyrosin oder L-Homoarginin.

X bedeutet einen oder zwei Reste von in Proteinen natürlich vorkommenden Aminosäuren, deren Seitenkettenfunktionen substituiert sein können, bevorzugt eine L-Aminosäure mit hydrophober Seitenkette, beispielsweise Phenylalanin, Tyrosin, Tryptophan, Leucin, Methionin und hydrophobe Derivate von natürlichen Aminosäuren, beispielsweise tertiär Butyl-serin.

Als Chromophor $H_2N$—R können zahlreiche Verbindungen dienen. Voraussetzung ist nur, daß das Fluoreszenz- oder Absorptionsspektrum von freiem und acyliertem Chromophor (in der —X—NH—R—Bindung) unter den Meßbedingungen hinreichend verschieden ist und eine klare quantitative Bestimmung des freien neben acyliertem Chromophor gestattet. Die in praxi verwendeten Verbindungen sind literaturbekannt. So kommen neben dem am häufigsten verwendeten einfachen 4-Nitranilin beispielsweise in Frage: 2- oder 3-Carboxy-4-nitranilin, 2- oder 3-Halogen-4-nitro-anilin, 2-Methoxy-4-nitro-5-methylanilin, 4-Nitranilin-2-sulfonsäure-Natriumsalz, 2-Nitro-4-trifluoromethylanalin, 2-Nitro-4-Cyanonitranilin, 4-Aminoazobenzol, 1-Aminonaphthalin-4-sulfonsäure, 2-Aminobenzimidazol, 2-Amino-pyrimidin, 4-Aminopyridin, 1-Aminotriazin, 3-Aminoindazol, 5-Nitro-2-aminobenzothiazol, 3-Amino-isophthalsäure-methylester, Aminopyren oder Aminocumarine oder deren Derivate.

Die erfindungsgemäßen Verbindungen werden nach den bekannten Methoden der Peptidchemie synthetisiert. Eine vorteilhafte Herstellungsweise ist beispielsweise die Kondensation eines Aminosäure- oder Peptidderivates der Formel III

$$W—A_{(1-4)}—OH \qquad (III)$$

worin W und $A_1$ bis $A_4$ die oben angegebene Bedeutung haben, wobei jedoch W kein Wasserstoffatom

bedeutet und Carboxy- und Aminogruppen in den Seitenketten von $A_1$—$A_4$ mit Schutzgruppen substituiert sind, mit einem Peptid der Formel IV

$$H—B—X—NH—R \quad (IV)$$

worin B, R und X die oben genannte Bedeutung besitzen, wobei jedoch Amino- und Carboxylgruppen in den Seitenketten der Aminosäurereste mit Schutzgruppen substituiert sind. Andere Seitenkettenfunktionen können substituiert sein. Danach können die Schutzgruppen gegebenenfalls abgespalten werden.

Zur Kondensation kommen die in der Peptidchemie üblichen Methoden in Frage, beispielsweise die Azidmethode oder die Methode der gemischten Anhydride. Bevorzugt verwendet man das bequeme Carbodiimidverfahren, gegebenenfalls unter Zusatz einer Verbindung wie beispielsweise 1-Hydroxybenzotriazol nach Chem. Ber. *103,* 788 (1970). Auch der Umsatz aktiver Ester mit der Aminkomponente kommt in Frage. Als Lösungsmittel für das Verfahren der gemischten Anhydride eignet sich beispielsweise Tetrahydrofuran. Für die anderen Methoden kommen bevorzugt Dimethylformamid, Dimethylacetamid, Dimethylsulfoxid oder N-Methylpyrrolidon in Frage.

In Tabelle 1 sind aus der großen Zahl der in Frage kommenden erfindungsgemäßen Verbindungen einige wenige chromogene Enzymsubstrate beispielhaft aufgeführt, die für die oben gegebene Beschreibung charakteristisch sind. Alle Verbindungen sind durch Elementaranalyse und Aminosäureanalyse charakterisiert worden. Sie stellen im besonderen den Gegenstand der Erfindung dar.

TABELLE 1
Chromogene Verbindungen

Z-Val-Arg-Val-pNa  pNa = p-Nitranilid
Boc-Ile-Glu(OBu$^t$)-Gly-Arg-Gly-pNa
Msc-Ile-Glu-Gly-Arg-Leu-pNa
Mes-Ile-Glu(OiPr)-Gly-Arg-Val-pNa; OiPR=Isopropylester
Tos-Ile-Glu(Morph)-Gly-Arg-Leu-pNa; Morph=Morpholid
Z-Val-Arg-Val-Phe-pNa
Etoc-Phe-Val-Arg-Phe-pNa
Meoc-Phe-Val-Arg-Phe-pNa
H-D-Phe-Val-Arg-Val-pNa
H-Gly-Pro-Gly-Gly-Arg-Leu-pNa
H-Gly-Pro-Glu-Gly-Arg-Leu-pNa
H-Gly-Pro-Lys(Ac)-Arg-Phe-pNa
H-B-Ala-Glu-Gly-Arg-Leu-pNa
Boc-D-Val-Leu-Lys-Leu-pNa
$CH_3$—$PEG_{600}$-CO-$(CH_2)_2$—CO-Val-Arg-Val-pNa $PEG_{600}$=Polyethylenglycol 600
Ac-Ile-Glu-Gly-Arg-Leu-pNa
Bz-Ile-Glu-Gly-Arg-Leu-pNa
Tos-Gly-Pro-Glu-Gly-Arg-Phe-pNa
H-Gly-Pro-Gly-Gly-Arg-Phe-pNa
H-Gly-Pro-Ala($SO_3$Na)-Gly-Arg-Phe-pNa
Msc-Gly-Gly-Pro-Glu(OiPr)-Gly-Arg-Phe-pNa
$C_4H_9$-CO-Ala-Glu-Gly-Gly-Gly-Val-Arg-Tyr-pNa
Ac-Cys(But)-Glu-Gly-Gly-Gly-Val-Arg-Phe-pNa
pGlu-Ile-Glu-Gly-Arg-Leu-pNa
For-Ile-Glu-Gly-Arg-Phe-pNa
H-D-Lys(Boc)-Pro-Arg-LeupNa
Msc-Cys(Bzl)-Val-Arg-Phe-pNa
TFA-D-Phe-Pro-Arg-Phe-pNa
Mal-D-Phe-Pro-Arg-Phe-pNa
Suc-D-Phe-Pro-Arg-Phe-pNa

In Tabelle 2 sind eine Reihe von Verbindungen der allgemeinen Formel IV aufgeführt. Sie stellen geeignete Ausgangsverbindungen zur Synthese der erfindungsgemäßen Substrate dar und enthalten sowohl die Spaltstelle wie auch das Chromogen.

TABELLE 2
Daten der Dipeptidnitranilide

H-Arg-(X)-pNa.2Y

| X | Y | Fp. °C (Z=Zersetzung) | $(\alpha)_D^{22}$ C=1 | Lös.-mittel | C | H (berechnet (%)) | N (berechnet (%)) | Halogen (%) |
|---|---|---|---|---|---|---|---|---|
| Gly | HCl | >134°(Z) | +14,1° | MeOH | 40,1 (39,6) | 5,7 (5,5) | 22,8 (23,1) | 15,9 (16,7) |
| Ala | HCl | >86°(Z) | −10,2° | MeOH | 41,2 (41,1) | 5,7 (5,7) | 22,1 (22,4) | 15,8 (16,2) |
| Val | HCl | >91°(Z) | +6,8° | MeOH | 43,5 (43,8) | 6,2 (6,3) | 20,8 (21,0) | 14,9 (15,2) |
| Leu | HCl | >115°(Z) | +13,3° | MeOH | 44,7 (45,0) | 6,5 (6,5) | 19,9 (20,4) | 14,3 (14,8) |
| Phe | HBr | >99°(Z) | +59,4° | MeOH | 41,5 (41,8) | 4,7 (4,8) | 16,2 (16,3) | 26,3 (26,5) |
| Tyr | HCl | >120°(Z) | +77,8° | MeOH | 45,3 (45,1) | 6,3 (6,4) | 13,3 (13,1) | 15,9 (16,6) |

H-Lys-(Msc)-(X)-pNa·Y

| X | Y | Fp. °C (Z=Zersetzung) | $(\alpha)_D^{22}$ C=1 | Lös.-mittel | C | H (berechnet (%)) | N (berechnet (%)) | S | Halogen (%) |
|---|---|---|---|---|---|---|---|---|---|
| Gly | HBr | 225—228°(Z) | +14,6° | MeOH | 38,8 (39,0) | 5,2 (5,1) | 12,3 (12,6) | 5,7 (5,8) | 14,6 (14,4) |
| Leu | HBr | >93°(Z) | −1,5° | MeOH | 42,4 (42,3) | 5,9 (5,9) | 11,3 (11,5) | 5,2 (5,3) | 13,4 (13,1) |
| Phe | HBr | >117°(Z) | +65,7° | MeOH | 46,3 (46,6) | 5,2 (5,3) | 11,0 (10,9) | 4,9 (5,0) | 12,6 (12,4) |
| Tyr | Hbr HBr | >132°(Z) | +61,8° | MeOH | 45,7 (45,5) | 5,1 (5,2) | 10,9 (10,6) | 5,0 (4,9) | 12,0 (12,1) |

Tabelle 3 führt einige erfindungsgemäße Verbindungen auf, bei denen unter Konstanthaltung der Komponente W—A—B—X—NH der chromogene Rest variiert wurde. Es handelt sich nicht um eine vollständige Aufzählung aller denkbaren Reste, sondern nur um einige Beispiele, welche die Erfindung diesbezüglich erläutern sollen.

## TABELLE 3

Chromogene Verbindungen der Formel Z—Val—Arg—Val—NH—R

R =
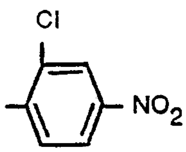

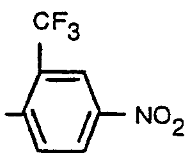

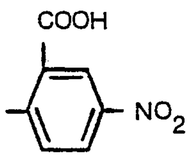

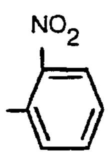

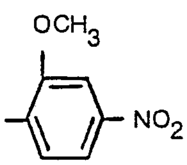

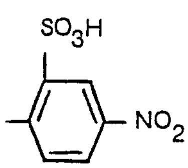

R =
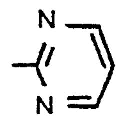

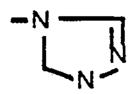

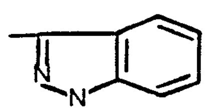

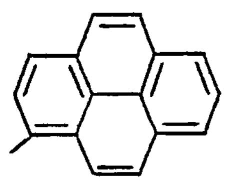

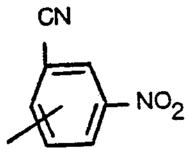

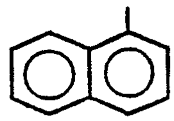

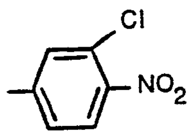

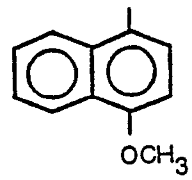

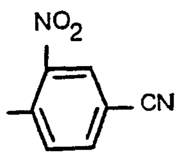

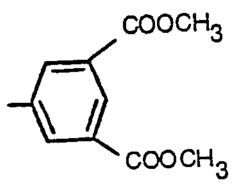

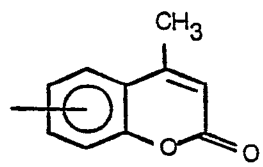

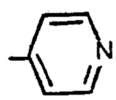

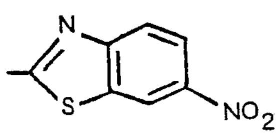

In Tabelle 4 sind die Spaltungsgeschwindigkeiten einiger erfindungsgemäßer Verbindungen durch verschiedene proteolytische Enzyme miteinander verglichen. Die Zahlen geben die Zeit in Minuten an, die notwendig ist, um aus 1,5 molaren Lösungen der Substrate durch die angegebenen Enzyme bei 405 nm eine Zunahme der optischen Dichte (OD) um 0,1 Einheit (E) zu erhalten.

Die Spaltungsgeschwindigkeiten sind durch die erhöhte Bindungsenergie der echten Peptidbindungen gegenüber der Nitroanilidbindung meist verringert. Durch die Variation von X läßt sich neben der Spezifität auch die Spaltgeschwindigkeit beeinflussen. Wegen der besser einstellbaren Spaltungsgeschwindigkeit (durch Variation von X) kann häufig auch mit höherer Enzymkonzentration gearbeitet werden; damit entfällt eine Mehrfachverdünnung als zusätzliche Fehlerquelle bei Enzymbestimmungen.

TABELLE 4

Vergleich der Spaltungsgeschwindigkeiten verschiedener Substrate durch proteolytische Enzyme (Zahlen geben die Zeit in Minuten an, die benötigt wird, um bei 405 nm eine Extinktionssteigerung von 0,1 E optische Dichte (OD) zu erreichen)

| Substrate 1,5 m mol/l | Thrombin 6 IU/ml | Plasmin 2 CTA-U/ml | F Xa 0,2 U/ml | Kallikrein 0,85 BAEE-U/ml | F XIIa 3,6 U/ml |
|---|---|---|---|---|---|
| **Substrate für Thrombin nach Stand der Technik:** | | | | | |
| H–D–Phe–Pip–Arg↓pNa | 0,4 | 2,0 | 6,3 | 0,5 | 0,3 |
| Tos–Gly–Pro–Arg↓pNa | 0,3 | 0,4 | 0,7 | 0,4 | 0,2 |
| **Erfindungsgemäße Substrate für Thrombin:** | | | | | |
| H–D–Phe–Pro–Arg↓Leu–pNa | 1,3 | 33,0 | 94,0 | 4,7 | 2,5 |
| H–D–Phe–Pro–Arg↓Val–pNa | 5,3 | --- | --- | 1,6 | 10,9 |
| **Substrate für Kallikrein nach Stand der Technik:** | | | | | |
| Bz–Pro–Phe–Arg↓pNa | --- | 3,4 | --- | 0,2 | 12,8 |
| H–D–Pro–Phe–Arg↓pNa | 6,9 | 0,4 | 3,5 | 0,11 | 0,5 |
| **Erfindungsgemäße Substrate für Kallikrein:** | | | | | |
| MSC–Val–Arg↓Val–pNa | --- | --- | --- | 1,8 | --- |
| Z–Val–Arg↓Val–pNa | --- | --- | --- | 1,4 | --- |
| Z–Val–Arg↓Phe–pNa | --- | --- | --- | 3,8 | --- |
| Boc–Gly–Pro–Glu– (O–But) Gly–Arg↓Phe–pNa | --- | --- | --- | 0,91 | --- |
| Boc–Ile–Glu (O–But) Gly–Arg↓Leu–pNa | --- | --- | 36,0 | 1,6 | --- |
| Boc–Gly–Pro–Leu–Gly–Arg↓Leu–pNa | --- | --- | --- | 0,5 | --- |
| Boc–D–Val–Glu– (O–But) Gly–Arg↓Leu–pNa | --- | --- | 5,6 | 1,4 | --- |
| Bz–Ile–Glu–Gly–Arg↓Phe–pNa | --- | --- | --- | 4,7 | --- |
| H–D–Phe–Glu–Gly–Arg↓Phe–pNa | --- | --- | --- | 2,0 | --- |
| H–D–Phe–Glu–Gly–Arg↓Leu–pNa | --- | --- | --- | 2,7 | --- |
| H–D–Ala–Glu–Gly–Arg↓Leu–pNa | --- | --- | --- | 3,4 | --- |

TABELLE 4 (Fortsetzung)

| Substrate 1,5 m mol /l | Thrombin 6 IU /ml | Plasmin 2 CTA-U /ml | F Xa 0,2 U /ml | Kallikrein 0,85 BAEE-U /ml | F XIIa 3,6 U /ml |
|---|---|---|---|---|---|
| Substrate für Plasmin nach Stand der Technik: | | | | | |
| H–D–Val–Leu–Lys↓–pNa | --- | 0,4 | --- | 0,5 | --- |
| Erfindungsgemäßes Substrate für Plasmin: | | | | | |
| Boc–D–Val–Leu–Lys↓–Leu–pNa | --- | 3,3 | --- | 15,0 | --- |

↓ Spaltstelle der angegebenen Substrate für Serinproteasen

--- keine nachweisbare Substratspaltung

Tabelle 5 enthält einige Beispiele der Spezifitätsänderung von Substraten durch Variation von X. Es zeigt sich, daß die Einführung der Gruppe X eine Vielzahl von neuen Möglichkeiten zur Beeinflussung der Spezifität eröffnet.

In Tabelle 6 werden die Quotienten der Spaltungsgeschwindigkeiten von Substraten nach dem Stand der Technik und erfindungsgemäßen Substraten mit Thrombin und acetyliertem Thrombin nach der Vorschrift von Landaburu und Seegers, Can. J. Biochem. Physiol. *37*, 1361 (1959), verglichen. Ein in dieser Weise acetyliertes Thrombin besitzt keine Gerinnungsaktivität mehr, zeigt aber eine ähnliche Esteraseaktivität wie vor der Acetylierung. Die Spaltung einer echten Peptidbindung in den erfindunggemäßen Substraten im Vergleich zu einer aktivierten Säureamidbindung in den Substraten nach dem Stand der Technik erlaubt eine bessere Differenzierung von nativen und partiell veränderten Proteasen, die ihre proteolytische Aktivität verloren haben, aber noch esterolytisch wirksam sind und führt zu einem beträchtlichen Spezifitätsgewinn.

Damit wird die Gefahr von Fehlinterpretationen der Ergebnisse von Proteasebestimmungen mit diesen Substraten wesentlich geringer als sie von Gaffney et al. in Thromb. Res. *10*, 549 (1977) für bisher bekannte chromogene Substrate beschrieben ist.

TABELLE 5

Spezifitätsänderung der Proteasesubstrate durch Aminosäureaustausche an der variablen Stelle X von Formel I (Zahlen geben die Zeit in Minuten an, die benötigt wird, um bei 405 nm eine Extinktionssteigerung von 0,1 E optische Dichte (OD) zu erreichen).

| Substrate 1,5 m mol/1 | Thrombin 6 IU/ml | Plasmin 2CTA-U/ml | F Xa 0,2 U/ml | Kallikrein 0,85 BAEE U/ml | F XIIa 3,6 U/ml U/ml |
|---|---|---|---|---|---|
| P-B-X-NH-R | | | | | |
| H-D-Phe-Pro-Arg↓-Leu-pNa | 1,3 | 33,3 | 94,0 | 4,7 | 2,5 |
| H-D-Phe-Pro-Arg↓-Phe-pNa | 0,6 | 9,6 | 2,1 | 5,6 | 2,9 |
| H-D-Phe-Pro-Arg↓-Tyr-pNa | 0,8 | 7,1 | 2,5 | 2,0 | 2,1 |
| H-D-Phe-Pro-Arg↓-Ser-pNa | — | — | — | — | — |
| Boc-D-Val-Glu(O-But)-Gly-Arg↓-Gly-pNa | — | — | — | — | — |
| Boc-D.Val-Glu(O-But)-Gly-Arg↓-Leu-pNa | — | — | — | 1,4 | — |
| Z-Arg↓-Ser(O-But)-pNa | — | — | — | 4,8 | — |
| Z-Arg↓-Val-pNa | — | — | — | 11,1 | — |
| Z-Arg↓-Gly-pNa | — | — | — | — | — |
| H-D-Val-Glu-Gly-Arg↓-Leu-pNa | — | — | — | 5,9 | — |
| H-D-Val-Glu-Gly-Arg↓-Ser-pNa | — | — | — | — | — |
| H-D-Val-Glu-Gly-Arg↓-Gly-pNa | — | — | — | — | — |
| Boc-D-Val-Leu-Lys↓-Tyr-pNa | — | 1,4 | — | 12,5 | — |
| Boc-D-Val-Leu-Lys↓-Leu-pNa | — | 3,3 | — | 15,0 | — |
| Boc-D-Val-Leu-Lys↓-Phe-pNa | — | — | — | — | — |
| Boc-D-Val-Leu-Lys↓-Gly-pNa | — | — | — | — | — |

↓ = Spaltstelle der angegebenen Substrate für Serinproteasen
— = keine meßbare Substratspaltung

TABELLE 6

Quotienten der Spaltungsgeschwindigkeiten von chromogenen Substraten vor und nach Acetylierung anhand von 2 Thrombin-Chargen

| Substrat | Charge 1 Q *) | Charge 2 Q *) |
|---|---|---|
| Substrate nach Stand der Technik: | | |
| Tos-Gly-Pro-Arg↓-pNa | 2,2 | 2,4 |
| Bz-Phe-Val-Arg↓-pNa | 3,1 | 2,5 |
| H-D-Phe-Pip-Arg↓-pNa | 1,4 | 1,3 |
| Erfindungsgemäße Substrate: | | |
| H-D-Phe-Pro-Arg↓-Leu-pNa | 7,0 | 5,6 |
| Boc-D-Phe-Pro-Arg↓-Tyr-pNa | 10,3 | 10,6 |
| Meoc-D-Phe-Pro-Arg↓-Leu-pNa | 9,0 | 6,8 |

$$*)\ Q = \frac{\text{milli Units/mg Protein vor Acetylierung}}{\text{milli Units/mg Protein nach Acetylierung}}$$

↓ = Spaltstelle der chromogenen Substrate durch Serinproteasen/esterasen

Die folgenden Beispiele erläutern zunächst die Herstellung der neuen Verbindungen; danach deren Verwendung zur Bestimmung von proteolytischen Enzymen.

Beispiel 1
Z-Val-Arg-Val-pNa

1.1 *Z-Val-pNa*

25,1 g (0,1 mol) Z-Val-OH, 13,8 g (0,1 mol) p-Nitranilin und 13,6 g (0,2 mol) Imidazol werden in 120 ml THF gelöst, die Lösung auf —20°C gekühlt und mit 9,3 ml (0,1 mol) Phosphoroxytrichlorid versetzt. Die Reaktionsmischung wird 24 Stunden bei Raumtemperatur gerührt, danach auf Eiswasser gegossen und mit Soda alkalisch gestellt. Die wässrige Lösung wird mehrfach mit Essigester extrahiert, die organische Phase wird über Natriumsulfat getrocknet und eingedampft. Der Rückstand wird aus heißem Alkohol unter Verwendung von Aktivkohle umkristallisiert.

Ausbeute: 18,9 g (51%)
Fp.: 192—194°C $(\alpha)_D^{22}$: —16,4° (c=1, Methanol)
Elementaranalyse: (371,38) $C_{19}H_{21}N_3O_5$
C 61,3 (61,44) H 5,55 (5,70) N 11,2 (11,31)

1.2. *H-Val-pNa-HBr*

11,8 g (32 mmol) Z-Val-pNa werden in 150 ml Eisessig suspendiert, 37,5 ml 40%ige HBr/Eisessig zugegeben und die Lösung 1 Stunde bei Raumtemperatur gerührt. Danach wird der Reaktionsansatz in 1,5 l Ether gegossen und der anfallende Niederschlag abgesaugt, gründlich mit trockenem Ether gewaschen und über KOH im Vakuum getrocknet.

Ausbeute: 7,7 g
Fp: 130°C (Zers.) $(\alpha)_D^{22}$: +52,7° (c=1, Methanol)
Elementaranalyse: (318,18) $C_{11}H_{16}BrN_3O_3$
C 41,4 (4162) H 5,0 (5,06) Br 24,9 (25,11) N 13,1 (13,2)

1.3. *H-Arg-Val-pNa·2 HCl*

7,3 g (23 mmol) H-Val-pNa·HBr, 7,1 g (23 mmol) Boc-Arg-OH·HCl und 3,1 g (23 mmol) HOBt werden in 70 ml Dimethylformamid gelöst, die Lösung auf 0°C gekühlt und mit 4,7 g (23 mmol) DCCI versetzt. Mit 8,7 ml N-Ethylmorpholin wird neutralisiert und 16 Stunden bei Raumtemperatur gerührt. Die Reaktionsmischung wird vom ausgefallenen Dicyclohexylharnstoff befreit, die klare Lösung eingedampft, der Rückstand zwischen $NaHCO_3$-Lösung und n-Butanol verteilt und die Butanolphase zur Trockne eingedampft. Der Rückstand wird über Kieselgel im Fließmittel $CHCl_3/CH_3OH/H_2O/HCOOH$ (400/100/10/3; v/v) chromatographiert. Die reine Dipeptidfraktion wird gesammelt, zur Trockne eingedampft, der Rückstand mit Ether verrieben, abgesaugt und im Vakuum über $P_2O_5$ getrocknet. Eine 50%ige wässrig-methanolische Lösung der Substanz wird mit dem Ionenaustauscher Amberlite IRA-410(C1-Form) behandelt und somit in das Hydrochlorid überführt.

Ausbeute: 9,3 g (≙76%).

Die so erhaltene Substanz wird nun in 100 ml 1,2 N HCl in Eisessig gelöst und 1 Stunde bei Raumtemperatur gerührt. Nach Abdampfen des Lösungsmittels wird der Rückstand mit Ether digeriert, filtriert, mit Ether gewaschen und über KOH im Vakuum getrocknet.

Ausbeute: 7,8 g (95,5%)

Fp: >91°C (Zers.) $(\alpha)_D^{22}$: +6,8° (c=1, Methanol)

1.4 *Z-Val-Arg-Val-pNa·HCl*

7,8 g (16,7 mmol) H-Arg-Val-pNa·2 HCl, 8,6 g (20 mmol) Z-Val-OTc und 2,7 g (20 mmol) HOBt werden in 75 ml Dimethylformamid gelöst und mit 6,5 ml N-Ethylmorpholin versetzt. Nach 16 Stunden Reaktionszeit wird das Lösungsmittel abgedampft und der verbleibende Rückstand mehrfach mit Petroether digeriert, wonach der Petroetherextrakt verworfen wird. Das Produkt wird nun zwischen $NaHCO_3$-Lösung und n-Butanol verteilt, die Butanolphase eingedampft und die so gewonnene Substanz über Kieselgel im Fließmittel $CHCl_3/CH_3OH/H_2O/HCOOH$ (400/100/10/1; v/v) chromatographiert. Die einheitliche Tripeptidfraktion wird gesammelt, zur Trockne eingedampft, mit Ether digeriert, filtriert und über $P_2O_5$ getrocknet.

Ausbeute: 7,0 g (63%)

Fp.: 152°C (Zers.) $(\alpha)_D^{22}$: −51,5° (c=1, Methanol)

Elementaranalyse: (663,17) $C_{30}H_{43}ClN_8O_7$

C 54,0 (54,33) H 6,5(6,54) N 16,8(16,89) Cl 5,2(5,38)

Die Verbindung ist als Substrat für die Bestimmung von Human-Plasma-Kallikrein geeignet.

## Beispiel 2

Boc-Gly-Pro-Glu(O-But)-Gly-Arg-Phe-pNa

2.1. *Z-Phe-pNa*

29,9 g (0,1 mol) Z-Phe-OH, 13,8 g (0,1 mol) Nitranilin und 13,6 g (0,2 mol) Imidazol werden in 150 ml Tetrahydrofuran gelöst und analog 1.1 mit $POCl_3$ umgesetzt.

Ausbeute: 29,6 g (70,5%)

Fp.: 132°C (Zers.) $(\alpha)_D^{22}$: +55,6° (c = 1, Methanol DMF, 1:1)

2.2. *H-Phe-pNa·HBr*

15 g (35,8 mmol) Z-Phe-pNa werden in 100 ml Eisessig suspendiert, 80 ml 36%iger HBr in Eisessig zugegeben und 1 Stunde bei Raumtemperatur gerührt. Die Mischung wird danach in 1,5 l trockenen Ether eingegossen, der Niederschlag abgesaugt, mit Ether gewaschen und über KOH im Vakuum getrocknet.

Ausbeute: 12,3 g (93,8%)

Fp.: 126—128°C $(\alpha)_D^{22}$: +77,7° (c=1, Dimethylformamid)

2.3. *H-Arg-Phe-pNa·2 HBr*

18,3 g (50 mmol) H-Phe-pNa·Br, 15,5 g (50 mmol) Boc-Arg-OH·HO und 6,75 g (50 mmol) HOBt werden in 150 ml Dimethylformamid gelöst, die Lösung wird auf 0°C abgekühlt und mit 10,3 g (50 mmol) DCCI und 13,9 ml N-Ethylmorpholin versetzt. Nach 16 Stunden Reaktionszeit wird der ausgefallene Niederschlag abfiltriert und das Filtrat zur Trockne eingedampft. Der Rückstand wird zwischen $NaHCO_3$-Lösung und Essigester verteilt, der Essigesterextrakt nach dem Eindampfen in Tetrahydrofuran gelöst und das Produkt mit Ether ausgefällt.

Ausbeute: 27,7 g (95%).

Die Substanz wird mit dem Ionenaustauscher Amberlite IRA 410 (Cl-Form) in das Hydrochlorid überführt.

$(\alpha)_D^{22}$: +10,8° (c=1, Methanol).

13,5 g (23,3 mmol) Boc-Arg-Phe-pNa·HCl werden in 100 ml 1 N HCl in Eisessig 1 Stunde bei Raumtemperatur gerührt, eingeengt, mit Ether gefällt und abgesaugt. Der Feststoff wird in wenig Methanol gelöst und mit 5 g Pyridinhydrobromid verrührt. Nach Zusatz von Ether wird der gebildete Niederschlag abgesaugt, mit Ether gründlich gewaschen und über KOH im Vakuum getrocknet.

Ausbeute: 12,4 g (=88%)

Fp.: 99°C (Zers.) $(\alpha)_D^{22}$: +59,4° (c=1, Methanol)

2.4. *Boc-Gly-Pro-OH*

27,6 g (78 mol) Boc-Gly-OTcp und 8,95 g Prolin (78 mmol) werden in 200 ml Dimethylformamid gelöst. Man gibt 10 ml N-Methylmorpholin (78 mmol) zu und hält über Nacht bei Raumtemperatur. Dann destilliert man das Lösungsmittel im Vakuum ab und verteilt den Rückstand zwischen 100 ml n-Butanol und 50 ml 10%igem $KHSO_4/K_2SO_4$ (1:1), extrahiert die wässrige Phase zweimal mit je 50 ml n-Butanol, wäscht die vereinigten Butanolphasen mit 20 ml 10%iger NaCl-Lösung und destilliert das Butanol im Vakuum ab. Der Rückstand wird in 50 ml Essigester gelöst. Man filtriert von etwas Salzen ab, engt die Essigesterlösung auf die Hälfte ein und fällt mit 100 ml Petrolether 17,5 g (82,5%) des chromatographisch reinen Peptids aus.

2.5. *Z-Glu(O-But)-Gly-OMe*

33,8 g (0,1 mol) Z-Glu(O-But)-OH, 12,6 g (0,1 mol) H-Gly-OMe, HCl, 13,5 g (0,1 mol) 1-Hydroxybenzotriazol und 26 ml (0,2 mol) N-Ethylmorpholin werden in 300 ml Dimethylformamid gelöst. Man gibt 22 g (106 mmol) Dicyclohexylcarbodiimid zu und rührt über Nacht bei Raumtemperatur. Nach Abfiltrieren von Dicyclohexylharnstoff wird das Lösungsmittel im Vakuum abdestilliert. Der Rückstand wird in 100 ml Essigester gelöst, die Lösung filtriert. Man schüttelt mit gesättigtem Hydrogencarbonat und 10%iger Citronensäure aus, wäscht mit wenig Wasser, trocknet die Essigesterlösung über Natriumsulfat und destilliert das Lösungsmittel im Vakuum ab. Es hinterbleibt ein Harz, das langsam durchkristallisiert.

Ausbeute an chromatographisch nahezu reiner Verbindung 37,1 g (91%).

2.6. *H-Glu(O-But)-Gly-OMe, TosOH*

13,2 g der Z-Verbindung werden in 100 ml Methanol unter Filtration mit 1N methanolischer TosOH an Pd katalytisch hydriert. Nach 30 min. ist die Reaktion beendet. Man filtriert vom Katalysator ab und destilliert Methanol im Vakuum ab. Der Rückstand wird an der Ölpumpe getrocknet.

Ausbeute 13,0 g Öl (91,5%). Nahezu chromatographisch rein.

2.7. *Boc-Gly-Pro-Glu(O-But)-Gly-OMe*

2,7 g (10 mmol) Boc-Gly-Pro-OH und 4,5 g (10 mmol) H-Glu(O-But)-Gly-OMe·TosOH werden in 20 ml Dimethylformamid gelöst. Man gibt 1,28 ml (10 mmol) N-Ethylmorpholin und 2,2 g (10,6 mmol) Dicyclohexylcarbidiimid zu und läßt über Nacht bei Raumtemperatur stehen. Dann wird vom Dicyclohexylharnstoff abfiltriert, das Lösungsmittel im Vakuum abdestilliert, der Rückstand in Essigester aufgenommen, die Lösung wie oben mit Citronensäure, Hydrogencarbonatlösung und Wasser gewaschen, über Natriumsulfat getrocknet und zur Trockne eingedampft. Der Rückstand wird in Äther gelöst und mit Petrolether ein Niederschlag ausgefällt, der abfiltriert wird.

Ausbeute: 2,9 g (55%) chromatographisch reiner Verbindung.

2.8 *Boc-Gly-Pro-Glu(O-But)-Gly-OH*

2,2 g (4,2 mmol) des Methylesters werden in einer Mischung aus 15 ml Dioxan, 5 ml Methanol und 7 ml Wasser gelöst. Man verseift bei pH 12,5 am Autotitrator. Nach Verbrauch von 4,6 ml 1N NaOH ist die Reaktion beendet. Man neutralisiert mit 1N HCl, destilliert die Lösungsmittel ab, verdünnt den Rückstand mit Wasser und gibt die restliche Menge 1N HCl bis zur Äquivalenz zu. Die trübe wässrige Lösung wird dreimal mit Essigester extrahiert, die vereinigten Essigesterlösungen mit wenig Wasser gewaschen, über Natriumsulfat getrocknet und im Vakuum zur Trockne eingeengt. Niederschlag aus Ether/Petrolether.

Ausbeute: 1,88 g (89%) der chromatographisch reinen Verbindung. $(\alpha)_D^{22}$: −65,2°C (c = 1, in Methanol. C, H, N: korrekt.

2.9. *Boc-Gly-Pro-Glu(O-But)-Gly-Arg-Phe-pNa·HBr*

514 mg (1 mmol) Boc-Gly-Pro-Glu(O-But)-Gly-OH, 603,5 mg (1 mmol) H-Arg-Phe-pNa·2 HBr und 135 mg (1 mmol) HOBt werden in 2 ml Dimethylformamid bei 0°C gelöst, mit 206 mg (1 mmol) DCCI und 0,38 ml N-Ethylmorpholin versetzt. Die Lösung wird 16 Stunden bei Raumtemperatur gerührt, der ausgefallene Niederschlag abgesaugt, das Filtrat zur Trockne eingedampft und der Rückstand zwischen Essigester und $NaHCO_3$-Lösung verteilt. Die über $Na_2SO_4$ getrocknete Essigesterphase wird eingedampft, der Rückstand in wenig Methanol gelöst und das Produkt mit Ether ausgefällt. Der Feststoff wird schließlich über Kieselgel im Fließmittel $CHCl_3/CH_3OH/H_2O/CH_3COOH$ (200/100/15/10; v/v) chromatographiert. Die reine Hexapeptidanilidfraktion wird gesammelt, eingedampft, mit wenig Methanol gelöst, 50 mg Pyridinhydrobromid dieser Lösung zugefügt, das Produkt durch Eintropfen von Ether in die kräftig gerührte Lösung ausgefällt und zentrifugiert.

Ausbeute: 146 mg; $(\alpha)_D^{22}$: −22,7° (c = 1, Methanol);
Aminosäurezusammensetzung Glu 0,93 Pro 0,8
Gly 1,95 Phe 0,96 Arg 1,0.
Die Verbindung ist als Substrat für die Bestimmung von Human-Plasma-Kallikrein geeignet.

## Beispiel 3
### Meoc-Ile-Glu-(O-But)-Gly-Arg-Phe-pNa

3.1. *Meoc-Ile-OH*

26,2 g (0,2 mol) Isoleucin werden in 100 ml 2N NaOH und 80 ml Wasser gelöst. Man tropft bei 0°C unter Vibromischung gleichzeitig 17 ml (0,22 mol) Chlorameisensäuremethylester und 116 ml 2N NaOH zu. 1 Stunde nach beendeter Zugaben wird mit Ether extrahiert, die wässrige Lösung mit konzen-

triertem HCl auf pH 2 gebracht und wieder mit Ether extrahiert. Die Etherlösung wird mit wenig 1N HCl und mit Wasser gewaschen, über Natriumsulfat getrocknet und zur Trockne eingedampft. Den Rückstand nimmt man in wenig Ether auf und fällt mit Petrolether 31,2 g Meoc-Ile-OH (82%) aus.

Fp.: 73°C $(\alpha)_D^{22}$: −3,0° (c = 1, in Methanol).

3.2. *Meoc-Ile-Glu(O-But)-Gly-OMe*

1,89 g (10 mmol) Meoc-Ile-OH und 4,5 g (10 mmol) H-Glu(O-But)-Gly-OMe, TosOH werden in 20 ml Dimethylformamid gelöst. Man gibt 1,28 ml (10 mmol) N-Ethylmorpholin und 2,2 g (10,6 mmol) Dicyclohexylcarbodiimid zu und rührt über Nacht. Nach Filtration wird das Lösungsmittel im Vakuum abdestilliert, der Rückstand in Essigester aufgenommen, wie oben mit Citronensäure, Natriumhydrogencarbonat und Wasser gewaschen, die Essigesterlösung über Natriumsulfat getrocknet und eingedampft. Der Rückstand wird mit wenig eiskaltem Essigester und Ether digeriert.

Ausbeute: 3,15 g (70,8%).

Fp.: 155—156°C.

$(\alpha)_D^{22}$: −42.8° (c = 1, in Methanol). C, H, N korrekt.

3.3. *Meoc-Ile-Glu(O-But)-Gly-OH*

2,9 g (5,9 mmol) Ester werden 30 ml Dioxan-Methanol-Wasser (30:20:5) mit 6,9 ml 1N NaOH bei pH 12,5 verseift (Autotitrator). Dann stellt man den pH mit 1,8 ml 1N HCl neutral, engt im Vakuum ein, gibt 5,1 ml 1N HCl zu und extrahiert mit Essigester. Die Essigesterlösung wird mit etwas Wasser gewaschen, über Natriumsulfat getrocknet und eingeengt. Das zurückbleibende Öl wird mit Ether fest.

Ausbeute: 1,95 g (76%).

Fp.: 110°C. $(\alpha)_D^{22}$: −44,7 (c = 1, in Methanol).

C, H, N korrekt.

3.4. *Meoc-Ile-Glu(O-But)-Gly-Arg-Phe-pNa·HCl*

648 mg (1,5 mmol) Meoc-Ile-Glu(O-But)-Gly-OH, 905 mg (1,5 mmol) H-Arg-Phe-pNa·2HBr und 205 mg (1,5 mmol) HOBt werden in 4,5 ml Dimethylformamid gelöst und auf 0°C gekühlt. Dieser Mischung werden 309 mg (1,5 m Mol) DCCI und 0,57 ml N-Ethylmorpholin zugefügt. Nach 16 Stunden Reaktionszeit wird vom ausfallenden Niederschlag abfiltriert, das Lösungsmittel abgedampft, das zurückbleibende Öl mit Ether verrieben, wobei ein Feststoff entsteht. Dieser wird abgesaugt, gründlich mit Ether gewaschen und durch zeimalige Chromatographie über LH 20/Methanol gereinigt.

Ausbeute: 575 mg;

$(\alpha)_D^{22}$: −11,2° (c = 1, Methanol)

Die Verbindung ist als Substrat für die Bestimmung des Gerinnungsfaktors X geeignet.

## Beispiel 4
## H-D-Phe-Pro-Arg-Phe-pNa

4.1. *Boc-D-Phe-Pro-Bzl*

13,43 g (55,5 mmol) H-Pro-OBzl·HCl und 14,72 g (55,5 mmol) Boc-D-Phe-OH werden in 70 ml DMF gelöst, die Lösung wird auf 0°C gekühlt und mit 7,1 ml N-Ethylmorpholin und 12,1 g (58,7 mmol) DCCI versetzt. Die Reaktionsmischung wird 3 Stunden bei 0°C und 4 Stunden bei Raumtemperatur gerührt, der Niederschlag wird abfiltriert und das Filtrat zur Trockne eingedampft. Der Rückstand wird in Essigester/Ether (2:1; v/v) aufgenommen und je dreimal mit Citronensäurelösung, $NaHCO_3$-Lösung und NaCl-Lösung ausgeschüttelt, die organische Phase wird über $Na_2SO_4$ getrocknet, eingeengt und über Kieselgel im Fließmittel $CHCl_3/CH_3OH$ (50/1, v/v) getrennt. Die reine Dipeptidfraktion wird gesammelt und eingedampft. Zurück bleibt eine ölige, dünnschichtchromatographische reine Substanz.

Ausbeute: 19 g (≙ 75%).

4.2. *Boc-D-Phe-Pro-OH*

19 g (41,9 mmol) Boc-D-Phe-Pro-OBzl werden in 400 ml Methanol gelöst und in Gegenwart von Pd/Aktivkohle im Verlauf von 4 Stunden hydriert. Der Katalysator wird durch Filtration über Kieselguhr abgetrennt, das Filtrat eingeengt und mit Petrolether ausgefällt.

Ausbeute: 14,2 g (93%).

Fp.: 168—171°C $(\alpha)_D^{22}$: −90,8° (c = 1, Methanol).

4.3. *H-D-Phe-Pro-Arg-Phe-pNa·$CF_3COOH$*

370,4 mg (1,02 mmol) Boc-D-Phe-Pro-OH, 615,4 mg 1,02 mmol) H-Arg-Phe-pNa·HBr und 137,9 mg (1,02 mmol) HoBt werden in 3 ml Dimethylformamid gelöst, die Lösung auf 0°C gekühlt und mit 230 mg (1,12 mmol) DCCI versetzt. Nach Zugabe von 0,13 ml N-Ethylmorpholin rührt man 1 Stunde bei 0°C und weitere 8 Stunden bei Raumtemperatur, filtriert den Niederschlag ab und dampft zur Trockne ein. Der Rückstand wird zwischen $NaHCO_3$-Lösung und n-Butanol verteilt, die Butanolphase mit $K_2CO_3$ getrocknet, eingeengt und das Produkt mit Ether ausgefällt.

Ausbeute: 504 mg (≙54%) $(\alpha)_D^{22}$: −32,3° (c = 1, Methanol.

Fp.: 160°C.

450 mg (0,44 mmol) Boc-D-Phe-Pro-Arg-Phe-pNa·HBr werden in 4 ml Trifluoressigsäure unter Zusatz von 0,1 ml Anisol gelöst und 45 min bei Raumtemperatur gerührt. Das Produkt wird mit Ether ausgefällt, zentrifugiert, mehrmals mit Ether verrieben und erneut zentrifugiert. Die getrocknete Substanz wird mit $NaHCO_3$-Lösung verrieben und mit n-Butanol extrahiert, die organische Phase wird mit $K_2CO_3$ getrocknet, filtriert und zur Trockne eingedampft. Der feste Rückstand wird mit Ether verrieben und abzentrifugiert.

Ausbeute: 320 mg;
Fp.: 156—158°C

Die Verbindung ist als Substrat für die Bestimmung von Thrombin bzw. Thrombininhibitoren geeignet.

Beispiel 5

Boc-D-Val-Leu-Lys-Leu-pNa

5.1 *Z-Lys(Msc)-Leu-pNa*

4 g (10 mmol) Z-Lys(Msc)-OH, 3,3 g (10 mmol) H-Leu-pNa·HBr und 1,35 g (10 mmol) HOBt werden bei 0°C in 45 ml Dimethylformamid gelöst, mit 2,06 g (10 mmol) DCCI und 2,5 ml N-Ethylmorpholin versetzt. Nach 20 Stunden Reaktionszeit bei Raumtemperatur wird der ausgefallene Niederschlag abfiltriert, das Filtrat eingeengt, das zurückbleibende Öl in Essigester aufgenommen, dreimal mit Phosphatpuffer (pH 7), zweimal mit $KHSO_4$-Lösung und zweimal mit Wasser ausgeschüttelt. Die über $Na_2SO_4$ getrocknete Essigesterphase wird eingedampft, der Rückstand mit Ether verrieben, abgesaugt und aus Essigester umkristallisiert.

Ausbeute: 4,85 g (76%);
Fp.: 159—161°C (zers.) $(\alpha)_D^{22}$: 7,4° (c = 1,
Dimethylformamid;
C, H, N, S: korrekt.

5.2. *H-Lys(Msc)-Leu-pNa·HBr*

4 g (6,2 mmol) Z-Lys(Msc)-Leu-pNa werden in 20 ml Essigester und 12 ml Eisessig gelöst und mit 8 ml 36%iger HBr in Eisessig erst 1 Stunde bei 0°C und dann 1 Stunde bei Raumtemperatur gerührt. Dann wird die Mischung eingeengt, mit Ether digeriert, der Feststoff abgesaugt, gründlich mit Ether gewaschen und über KOH im Vakuum getrocknet.

Ausbeute: 2,5 g (67%);
Fp.: 225—228°C; $(\alpha)_D^{22}$: +14,6 (c = 1, Methanol)

5.3. *Boc-D-Val-Leu-OH*

10,86 g (50 mmol) Boc-D-Val-OH 9,08 g (50 mmol) H-Leu-OMe·HCl und 6,75 g (50 mmol) HOBt werden in 200 ml Dimethylformamid gelöst. Die Lösung wird auf 0°C gekühlt, mit 10,3 g (50 mmol) DCCI und 12,5 ml N-Ethylmorpholin versetzt. Die Lösung wird 20 Stunden bei Raumtemperatur gerührt, danach wird der Niederschlag abgesaugt und das Filtrat zur Trockne eingedampft. Das zurückbleibende Öl kristallisiert beim Behandeln mit Ether und Petrolether.

Ausbeute: 11,5 g (66,7%);
Fp.: 86—88°C; $(\alpha)_D^{22}$: −15,4° (c = 1, Dimethylformamid).

11,4 g (33 mmol) Boc-D-Val-Leu-OMe werden in 80 ml Dioxan und 10 ml Wasser gelöst, protionsweise mit der berechneten Menge 1 N NaOH versetzt und 2 Stunden bei Raumtemperatur gerührt. Danach wird mit $KHSO_4$ angesäuert, mit 300 ml Essigester überschichtet und ausgeschüttelt. Die über $Na_2SO_4$ getrocknete Essigesterphase wird eingeengt und das Produkt unter Zusatz von Petrolether kristallisiert.

Ausbeute: 10,3 (≙94%).
Fp.: 144—146°C; $(\alpha)_D^{22}$: −9,8° (c = 1, Dimethylformamid).

5.4 *Boc-D-Val-Leu-Lys(Msc)-Leu-pNa*

1,93 g (3,3 mmol) H-Lys(Msc)-Leu-pNa·HBr, 1,09 g (3,3 mmol) Boc-D-Val-Leu-OH, 0,45 g (3,3 mmol) HOBt bei 0°C in 10 ml Dimethylformamid werden mit 0,68 g (3,3 mmol) DCCI, 0,8 ml N-Ethylmorpholin versetzt und 16 Stunden bei Raumtemperatur gerührt. Nach dem Absaugen des Dicyclohexylharnstoffs wird das Filtrat eingedampft, der Rückstand in Essigester/n-Butanol (1/1; v/v) aufgenommen, mit pH 7-Puffer, Wasser, $KHSO_4$-Lösung und Wasser der Reihe nach ausgeschüttelt und über $Na_2SO_4$ getrocknet. Das Lösungsmittel wird abgedampft und der Rückstand mit Ether verrieben, filtriert, mit Ether gewaschen und im Vakuum über $P_2O_5$ getrocknet.

Ausbeute: 2,2 g (≙79%);
Fp.: 188—190°C (Zers.); $(\alpha)_D^{22}$: −7,2° (c = 1, Dimethylformamid).

5.5. *Boc-D-Val-Leu-Lys-Leu-pNa·HCl*

1,6 g (1,9 mmol) Boc-D-Val-Leu-Lys(Msc)-Leu-pNa werden in 40 ml Methanol gelöst, die Lösung auf 0°C gekühlt, 40 ml NaOH (2 N) zugegeben und nach 5 min mit der gleichen Menge Salzsäure neutralisiert. Die Lösung wird zur Trockne eingedampft, der Rückstand mit Methanol extrahiert und der Methanolextrakt über LH 20 mit Methanol als Fließmittel chromatographiert. Die reine Tetrapeptidfraktion wird gesammelt, eingedampft, der Rückstand mit Ether verrieben und abgesaugt.

Ausbeute: 880 mg (60%);
Fp.: 210—212°C (Zers.); $(\alpha)_D^{22}$: −35,9° (c = 1, Methanol).
Val 0,95 Leu 2,0 Lys 0,95

Die Verbindung ist als Substrat für die Bestimmung von Plasmin bzw. Plasmininhibitoren geeignet.

Beispiel 6

Z-Ser-Arg-Leu-Phe-pNa

6.1. *Z-Ser(O-But)-Arg-Leu-Phe-pNa·HBr*

1,02 g (1,8 mmol) Z-Ser(O-But)-Arg-Leu-OH (hergestellt nach Chem-Ber.*107*, 215—231 (1974)), 660 mg (1,8 mmol) H-Phe-pNa·HBr· und 243 mg (1,8 mmol) HOBt werden bei 0°C in 10 ml Dimethylformamid gelöst, der Lösung 370 mg (1,8 mmol) DCCI und 0,25 ml N-Ethylmorpholin zugesetzt und die Reaktionsmischung 16 Stunden bei Raumtemperatur gerührt. Der ausgefallene Niederschlag wird abgesaugt, das Filtrat zur Trockne eingedampft, der Rückstand in wenig Methanol aufgenommen und das Produkt mit Ether ausgefällt. Der Feststoff wird erneut in Methanol gelöst und nun mit $NaHCO_3$-Lösung ausgefällt. Der Niederschlag wird schließlich in Methanol gelöst und über eine LH 20-Säule mit Methanol als Fließmittel chromatographiert. Die gereinigte Tetrapeptidanilidfraktion wird gesammelt.

Ausbeute: 868 mg (53%); $(\alpha)_D^{22}$: −26,2° (c = 1, Methanol)

6.2. *Z-Ser-Arg-Leu-Phe-pNa·HCl*

100 mg (0,11 mmol) Z-Ser(O-But)-Arg-Leu-Phe-pNa·HBr werden in 5 ml 1N HCl in Eisessig gelöst und 1 Stunde bei Raumtemperatur gerührt, eingeengt und das Produkt mit Ether ausgefällt. Nach erneutem Umfällen aus Methanol/Ether und Trocknen im Vakuum über KOH erhält man 72 mg Tetrapeptidanilid.

$(\alpha)_D^{22}$: −21,6° (c = 1, Methanol);
Ser 0,88 Leu 1,0 Phe 0,95 Arg 0,94

Die Verbindung ist als Substrat für die Bestimmung von Gerinnungsfaktor X geeignet.

Beispiel 7

*Kallikrein-Bestimmung aus Plasma*

Das Präkillikrein in Plasma wird über die systemische Aktivierung des Gerinnungsfaktors XII (Hagemanfaktor) bestimmt, der in bekannter Weise Präkallikrein zu Kallifrein aktiviert. Zur enzymatischen Bestimmung des Kallikreins kann beispielsweise das Substrat Boc-Gly-Pro-Leu-Gly-Arg-Leu-pNa unter Mitwirkung der Aminopeptidase verwendet werden.

Testansatz zur Bestimmung von Präkallikrein aus Plasma
20 µl Plasma
700 µl Puffer
100 µl Faktor XII-Aktivator*
60 sec Inkubation bei 37°C
20 µl Aminopeptidase
200 µl Substrat (1,5 mM/l)

*Auswertung:* ΔOD/min × 5306.12 = milli-Units/ml Plasma
= Units/l Plasma

* PTT-Reagenz, klar der Behringwerke AG

Beispiel 8

*Prothrombin-Bestimmung aus Plasma*

Der Prothrombin-Gehalt in Plasma wird beispielsweise über eine systemische Aktivierung mit Gewebsthromboplastin (Thromborel®-Behringwerke) bestimmt. An der Aktivierungskaskade sind ähnlich wie bei der Aktivierung unter physiologischen Bedingungen die Gerinnungsfaktoren VII, X und V des "extrinsic"-Systems beteiligt. Gegenüber dem Stand der Technik, nach dem Prothrombin beliebiger Genese (auch cumarininduziertes Prothrombin) mit Ecarin (Eschiscarinatus-Schlangengift) aktiviert wird, erfaßt das hier vorgeschlagene system verändertes Prothrombin, wie es durch eine Behandlung mit Cumarin-derivaten (orale Antikoagulantien-Therapie) entsteht, nicht. Wegen der Spezifität der erfindungsgemäßen Substrate stören die enzymatischen Faktoren der Kaskade, beispielsweise F Xa, die Bestimmung nicht. Als Substrate können beispielsweise verwendet werden: H-D-Phe-Pro-Arg-Leu-pNa oder MeOC-D-phe-Pro-Arg-Leu-pNa.

Testansatz zur Prothrombin-Bestimmung aus Plasma
10 µl Plasma
200 µl Gewebsthromboplastin
3 min Inkubation bei 37°C
700 µl Puffer pH = 8,4
20 µl Aminopeptidase

150 µl Substrat (z.B. (B) 3 mM/1)

*Auswertung:* ΔOD/min × 5561.2 = milli-Units/ml Plasma
Units/1 Plasma

**Patentansprüche**

1. Verbindungen der allgemeinen Formel I

W—P—B—X—NH—R (I)

worin

W ein Wasserstoffatom, eine Acylgruppe oder eine Benzolsulfonyl-oder Alkarylsulfonylgruppe mit 7—10 Kohlenstoffatomen,

P einen Rest einer Aminosäure oder eines Di- bis Tetrapeptids, deren Seitenkettenfunktionen mit in der Peptidchemie üblichen Schutzgruppen substituiert sein können,

B Arginin, Lysin, Phenylalanin, Tyrosin oder Homoarginin,

X eine L-Aminosäure mit hydrophober Seitenkette oder ein hydrophobes Derivat einer natürlichen Aminosäure, und

R einen gegebenenfalls substituententragenden aromatischen Kohlenwasserstoffrest bedeutet, wobei X—NH—R eine chromogene Gruppe darstellt und die Eigenschaft besitzt, unter der hydrolytischen Einwirkung eines Enzyms ein Spaltprodukt $NH_2R$ abzugeben, dessen Menge durch photometrische, spektrophotometrische oder fluorenzphotometrische Methoden meßbar ist.

2. Verbindungen nach Anspruch 1, wobei W eine $R_1$-CO-Gruppe darstellt, worin $R_1$ ein aliphatischer Kohlenwasserstoffrest von 1—6 Kohlenstoffatomen, ein aromatischer Kohlenwasserstoffrest mit 6—10 Kohlenstoffatomen, ein araliphatischer Kohlenwasserstoffrest mit 7—10 Kohlenstoffatomen, eine Alkaryl-Gruppe mit bis zu 10 Kohlenstoffatomen, eine $R_2$—O—CO-Gruppe, in welcher $R_2$ einen aliphatischen Kohlenwasserstoffrest mit 1—6 Kohlenstoffatomen, einen araliphatischen Kohlenwasserstoffrest mit 7—10 Kohlenstoffatomen oder eine Alkyl$_{1-6}$-sulfonalkyl$_{2-6}$-Gruppe bedeutet, eine Benzolsulfonyl-Gruppe oder eine Alkarylsulfonyl-Gruppe mit 7—10 Kohlenstoffatomen ist.

3. Verbindungen nach Anspruch 1, wobei R eine Nitrophenyl-, Naphthyl-, Nitronaphthyl-, Methoxynaphthyl-, Phenyl-, Methylnitrophenyl-, Dinitrophenyl-, Chinolyl- oder Nitrochinolyl-Gruppe sowie deren Salze darstellt.

4. Verbindungen nach Anspruch 1, worin R ein Nitrophenylrest, der mit einem Halogenatom, einer Trifluormethylgruppe, einer Carboxygruppe, einer Methoxygruppe, einer Sulfosäuregruppe oder einer Cyanogruppe substituiert sein kann, eine Dimethoxycarbonyl-phenyl-Gruppe, ein 4-Azobenzol-4-Sulfonaphthalin-, Pyridin-, Nitrobenzothiazol-, Pyrazin-, Triazol-, Benzopyrazol-, Chrysen-, Naphthalin-, Methoxynaphthalin-, Azulen- oder Aminocumarinrest ist.

5. Verwendung einer Verbindung nach einem der Ansprüche 1 bis 4 zum Nachweis oder zur quantitativen Bestimmung eines proteolytischen Enzyms oder Enzyminhibitors.

6. Verfahren zur Bestimmung eines hydrolytisch wirksamen Enzyms, dadurch gekennzeichnet, daß man die Lösung, in der das Enzym bestimmt werden soll, versetzt mit einer Verbindung der allgemeinen Formel I in Anspruch 1, worin

W ein Wasserstoffatom, eine Acylgruppe oder eine Benzolsulfonyl-oder Alkarylsulfonylgruppe mit 7—10 Kohlenstoffatomen,

P einen Rest einer Aminosäure oder eines Di- bis Hexapeptids, deren Seitenkettenfunktionen mit in der Peptidchemie üblichen Schutzgruppen substituiert sein können, oder eine Bindung,

B Arginin, Lysin, Phenylalanin, Tyrosin oder Homoarginin,

X eine oder zwei L-Aminosäuren mit hydrophober Seitenkette oder ein hydrophobes Derivat einer natürlichen Aminosäure, und

R einen gegebenenfalls substituententragenden aromatischen Kohlenwasserstoffrest bedeutet, wobei X—NH—R eine chromogene Gruppe darstellt und die Eigenschaft besitzt, unter der hydrolytischen Einwirkung eines Enzyms ein Spaltprodukt $NH_2$—R abzugeben, dessen Menge durch photometrische, spektrophotometrische oder fluorenzphotometrische Methoden meßbar ist, und einem Enzym mit Aminopeptidase-Wirkung und nach einer Inkubationszeit das Spaltprodukt $NH_2R$ der Verbindung der allgemeinen Formel I mißt.

7. Verfahren nach Anspruch 6, dadurch gekennzeichnet, daß die Verbindung eine Verbindung nach einem der Ansprüche 1 bis 4 ist.

**Claims**

1. Compounds of the general formula I

W—P—B—X—NH—R (I)

wherein

W denotes a hydrogen atom, an acyl group or a benzene sulfonyl or alkarylsulfonyl group of 7—10 carbon atoms,

P denotes a radical of an amino acid or of a di- to tetrapeptide, the side chain functional groups of which can be substituted with protecting groups usual in peptide chemistry,

B denotes arginine, lysine, phenylalanine, tyrosine or homoarginine,

X denotes an L-amino acid with a hydrophobic side chain or a hydrophobic derivative of a naturally occurring amino acid,

and

R denotes an optionally substituted aromatic hydrocarbon radical, and wherein X—NH—R represents a chromogenic group and has the property of providing, under the hydrolytic action of an enzyme, a splitting product $NH_2$—R, the amount of which can be measured by photometric, spectrophotometric or fluorescencephotometric methods.

2. Compounds as claimed in claim 1, wherein W represents an $R_1$—CO-group, wherein $R_1$ denotes an aliphatic hydrocarbon radical with 1—6 carbon atoms, an aromatic hydrocarbon radical with 6—10 carbon atoms, an araliphatic hydrocarbon radical with 7—10 carbon atoms or an alkaryl group with up to 10 carbon atoms, an $R_2$—O—CO-group, in which $R_2$ denotes an aliphatic hydrocarbon radical with 1—6 carbon atoms, an araliphatic hydrocarbon radical with 7—10 carbon atoms or an $alkyl_{1-6}$-$sulfonylalkyl_{2-6}$ group, a benzenesulfonyl group or an alkarylsulfonyl group with 7—10 carbon atoms.

3. Compounds as claimed in claim 1, wherein R represents a nitrophenyl, naphthyl, nitronaphthyl, methoxynaphthyl, phenyl, methylnitrophenyl, dinitrophenyl, quinolyl or nitroquinolyl group and salts thereof.

4. Compounds as claimed in claim 1, wherein R is a nitrobenzene radical, which can be substituted by a halogen atom, a trifluoromethyl group, a carboxyl group, a methoxyl group, a sulfonic acid group or a cyano group, a dimethoxycarbonylphenyl group or a 4-azobenzene-4-sulfonaphthalene, pyridine, nitrobenzothiazole, pyrazine, triazole, benzopyrazole, chrysene, naphthalene, methoxynaphthalene, azulene or aminocoumarin radical.

5. Use of a compound as claimed in any of claims 1 to 4 for the detection or quantitative determination of a proteolytic enzyme or enzyme inhibitor.

6. Process for the determination of a hydrolytically active enzyme, which comprises adding a compound of the general formula I in claim 1, wherein

W denotes a hydrogen atom, an acyl group or a benzene sulfonyl or alkarylsulfonyl group of 7—10 carbon atoms,

P denotes a radical of an amino acid or of a di-to tetrapeptide, the side chain functional groups of which can be substituted with protecting groups usual in peptide chemistry, or denotes a bond,

B denotes arginine, lysine, phenylalanine, tyrosine or homoarginine,

X denotes one or two L-amino acids with a hydrophobic side chain or a hydrophobic derivative of a naturally occurring amino acid, and

R denotes an optionally substituted aromatic hydrocarbon radical, and wherein X—NH—R represents a chromogenic group and has the property of providing, under the hydrolytic action of an enzyme, a splitting product $NH_2$—R, the amount of which can be measured by photometric, spectrophotometric or fluorescencephotometric methods, and an enzyme with an aminopeptidase activity to the solution in which the enzyme is to be determined and, after an incubation period, measuring the product $NH_2R$ resulting from the cleavage of the compound of the general formula I.

7. Process as claimed in claim 6, which comprises that the compound is a compound as claimed in any one of claims 1 to 4.

## Revendications

1. Composés de formule I:

$$W—P—B—X—NH—R \qquad (I)$$

dans laquelle

W désigne un atome d'hydrogène, un groupe acyle ou un groupe benzènesulfonyle ou alcarylsulfonyle en $C_7$ à $C_{10}$,

P désigne un radical d'un aminoacide ou d'un di- à tétrapeptide, dont les fonctions des chaînes latérals peuvent être substituées par des groupes protecteurs habituels dans la chimie des peptides,

B désigne l'arginine, la lysine, la phénylalanine, la tyrosine ou l'homoarginine,

X désigne un L-aminoacide avec une chaîne latérale hydrophobe ou un dérivé hydrophobe d'un aminoacide naturel et

R désigne un radical hydrocarboné aromatique portant le cas échéant des substituants, X—NH—R représentant un groupe chromogène ou fluorogène, et possédant la propriété de céder sous l'action hydrolytique d'une enzyme, un produit de scission $NH_2$—R, dont la quantité est mesurable par des méthodes photométriques, spectrophotométriques ou de photométrie de fluorescence.

2. Composés suivant la revendication 1, dans lesquels W représente un groupe $R_1$—CO, où $R_1$ est un radical hydrocarboné aliphatique en $C_1$ à $C_6$, un radical hydrocarboné aromatique en $C_6$ à $C_{10}$, un radical hydrocarboné araliphatique en $C_7$ à $C_{10}$, un groupe alcaryle ayant jusqu'à 10 atomes de carbone, un groupe $R_2$—O—CO, dans lequel $R_2$ désigne un radical hydrocarboné aliphatique en $C_1$ à $C_6$, un radical hydrocarboné araliphatique en $C_7$ à $C_{10}$ ou un groupe alcoyl$_{1-6}$-sulfonalcoyle$_{2-6}$, un groupe benzène-sulfonyle ou un groupe alcarylsulfonyle en $C_7$—$C_{10}$.

3. Composés suivant la revendication 1, dans lesquels R désigne un groupe nitrophényle, naphthyle, nitronaphthyle, méthoxynaphthyle, phényle, méthylnitrophényle, dinitrophényle, quinoléyle ou nitroquinoléyle, ainsi que leurs sels.

4. Composés suivant la revendication 1, dans lesquels R désigne un radical nitrophényle qui peut être substitué par un atome d'halogène, un groupe trifluorométhyle, un groupe carboxy, un groupe méthoxy, un groupe acide sulfonique ou un groupe cyano, un groupe diméthoxy-carbonyl-phényle, un groupe 4-azobenzène-4-sulfonaphtalène, pyridine, nitrobenzothiazole, pyrazine, triazole, benzopyrazole, chrysène, naphtalène, méthoxynaphtalène, azulène ou aminocoumarine.

5. Utilisation d'un composé suivant l'une des revendications 1 à 4 pour la détection ou la détermination quantitative d'une enzyme protéolytique ou d'un inhibiteur d'enzyme.

6. Procédé de dosage d'une enzyme à activité hydrolytique, caractérisé en ce qu'on additionne la solution dans laquelle l'enzyme doit être dosée d'un composé de formule générale I de la revendication 1, dans laquelle

W représente un atome d'hydrogène, un groupe acyle ou un groupe benzènesulfonyle ou alcarylsulfonyle en $C_7$ à $C_{10}$,

P représente un radical d'un aminoacide ou d'un di- à hexapeptide, dont les fonctions des chaînes latérales peuvent être substituées avec des groupes protecteurs habituels dans la chimie des peptides, ou une liaison,

B représente l'arginine, la lysine, la phénylalanine, la tyrosine ou l'homoarginine,

X représente un ou deux L-aminoacides avec une chaîne latérale hydrophobe ou un dérivé hydrophobe d'un aminoacide naturel, et

R représente un radical hydrocarboné aromatique portant le cas échéant des substituants, X—NH—R représentant un groupe chromogène et possédant la propriété de céder, sous l'action hydrolytique d'une enzyme, un produit de coupure $NH_2$—R, dont la quantité est mesurable par des méthodes photométriques, spectrophotométriques ou par des méthodes de photométrie de fluorescence, et d'une enzyme à action d'aminopeptidase, et en ce qu'on dose après un temps d'incubation le produit de coupure $NH_2R$ du composé répondant à la formule générale I.

7. Procédé suivant la revendication 6, caractérisé en ce que le composé est un composé suivant l'une des revendications 1 à 4.